# EUROPEAN PATENT SPECIFICATION

(11) **EP 1 560 878 B1**
(45) Date of publication and mention of the grant of the patent: **17.05.2006**
(21) Application number: 03778349.5
(22) Date of filing: 11.11.2003
(51) Int. Cl.: C08K 5/3462, C07D 239/545, C07D 239/56, C08K 5/00, C08L 27/06

(54) **THERMALLY STABILISED PVC COMPOSITIONS**
HITZESTABILISIERTE PVC-ZUSAMMENSETZUNGEN
COMPOSITIONS DE PVC STABILISEES THERMIQUEMENT

(30) Priority: 15.11.2002 IT VA20020058
(43) Date of publication of application: 10.08.2005
(73) Proprietor: Lamberti Spa, 21041 Albizzate (IT)
(72) Inventor: CASIRAGHI, Angelo, I-20131 Milano (IT); NORCINI, Gabriele, I-21020 Comabbio (VA) (IT); ALLIERI, Guido, I-21050 Gorla Maggiore (VA) (IT); FERRACINI, Mauro, I-21010 Ferno (VA) (IT); MENEGUZZO, Enzo, I-21018 Sesto Calende (VA) (IT); VISCONTI, Marco, I-21100 Varese (IT); LI BASSI, Giuseppe, I-21026 Gavirate (VA) (IT)
(86) International application number: PCT/EP2003/050813
(87) International publication number: WO 2004/046236

(56) References cited:
- GB-A- 2 318 118
- US-A- 4 352 903
- US-A- 5 925 696

## Description

The present invention relates to rigid or flexible PVC compositions exhibiting high thermal stability and containing aminouracyls bearing a phenate or carboxylate group.

It is a fundamental object of the present invention a new class of substituted aminouracyls capable of imparting stability against thermal and oxidative decomposition of chlorine-containing polymers and in particular of PVC.

Said compounds are useful when used as stabilisers in rigid and flexible PVC compositions allowing the preparation of fully transparent stabilised system, without staining or blistering.

The behaviour of the compositions of rigid or flexible PVC containing the functionalised aminouracyls of the present invention is the more unexpected because these compositions, unlike the known stabilised compositions, do not need the addition of any metal soap.

In the present text with the expression "flexible PVC" we mean a composition containing PVC (PVC-S, PVC-E, PVC-M) and at least 5 phc of a plasticiser chosen among those commonly used; with the expression "rigid PVC" we mean a composition containing PVC (PVC-S, PVC-E, PVC-M) and less than 5 phc of the plasticizer.

In prior art, i.e. in patents DE 1694873, US 4,656,209, US 5,925,696, GB (A) 2,318,188A, EP (A1) 967 209, EP (A1) 967 245, EP (A1) 967208, EP (A1) 1 174 461 aminouracyls and aminothiouracyls are described as well as their use as PVC stabilisers; these patents neither describe the use of aminouracyls and aminothiouracyls bearing another functionality able to strongly increase the stabilising efficiency of the composition, nor describe the new aminouracyls of the present invention containing a phenate or carboxylate group.

According to a fundamental aspect of the present invention, the PVC compositions with high thermal stability contain aminouracyls functionalised with a phenate or carboxylate group and having formula I: 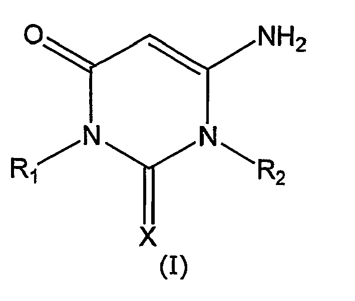 wherein:
X is O or S;
R₁ is a group of formula II: 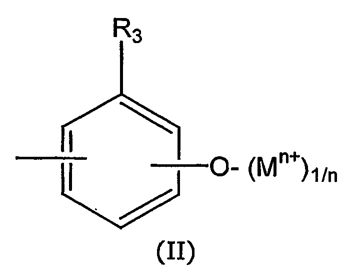
or R₁ is a group of formula III: 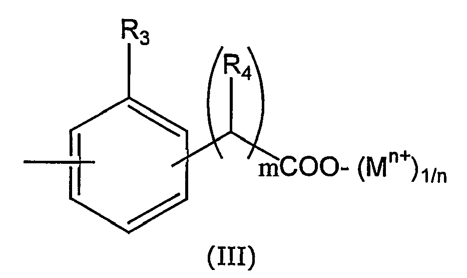
or R₁ is a group of formula IV: 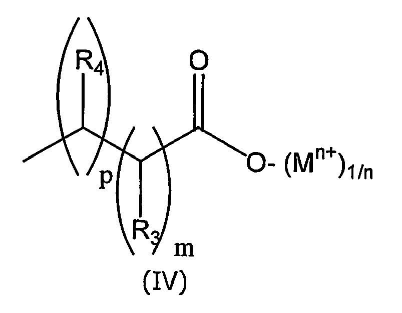 wherein Mⁿ⁺ is a metal cation of the II group of the periodic table or is Zn⁺⁺;
R₃ and R₄ can be the same or different and each independently of the other is selected from the group consisting of -H, C₁-C₆ linear o branched alkyl, -OH, C₁-C₆ linear or branched alkoxyl, halogen atom, -SH, C₁-C₆ linear or branched thioalkoxyl, NR₅R₆ (wherein R₅ and R₆ can be equal or different and are H or C₁-C₆ linear o branched alkyl), nitro, carboxyl, C₁-C₆ linear or branched carboxyalkyl;
m and p are integers having a value of from 0 to 6, with the proviso that they have not together the value of 0;
R₂ is the same as R₁ or it is selected in the group consisting of C₁-C₁₈ linear or branched alkyl, C₁-C₁₈ linear or branched alkyl substituted with -OH, halogen atom, C₁-C₆ linear or branched carboxyalkyl, -SH, C₁-C₆ linear or branched thioalkoxyl, NR₅R₆ (wherein R₅ and R₆ can be equal or different and are H or C₁-C₆linear o branched alkyl), C₁-C₆ linear or branched carboxyalkyl.

Preferably, the PVC compositions with a high thermal stability of the invention contain aminouracyls functionalised with a phenate or carboxylate group of formula I wherein M is selected among Ca, Ba and Zn, and n is 2.

Particularly suitable for the realisation of the present invention are PVC compositions containing aminouracyls functionalised with a phenate or carboxylate group and having formula I wherein:
R₂ is a C₁-C₁₈ linear or branched alkyl, R₁ is a group of formula II and R₃ is H; or
R₁ is a group of formula III, m is 0 and R₃ is H;
   or
R₁ is a group or formula IV and R₃ and R₄ are H.

The functionalised aminouracyls according to the present invention are normally obtained by cyclisation of a substituted urea using cyanoacetic acid or its derivatives.

According to the present invention, in flexible PVC compositions with a high thermal stability, the oligomeric and/or polymeric aminouracyls may be incorporated in an amount of from 0.01 to 10% by weight, preferably of from 0.05 to 5% and especially of from 0.1 to 3% based on the entire composition.

According to the present invention, stabilized PVC compositions can further contain customary additives such as, but not exclusively: stabilisers, auxiliaries and processing aids, compounds containing alkali metal compounds and alkaline earth metal compounds, lubricants, plasticisers, pigments, fillers, phosphites, thiophosphites, thiophosphates, mercaptocarboxylates, epoxidised fatty acid esters, antioxidants, UV absorbers and light stabilisers, fluorescent whitening agents, impact modifiers, chelant and antistatic agents, metals deactivators, flame retardants, blowing agents and antifogging agents.

Examples of plasticisers that can be used according to the present invention are: esters of C₄-C₂₀ alcohols (adipates, phthalates, trimellitates, azelates, sebacates, benzoates, phosphates), epoxidised compounds (typically epoxidised soy-bean oil), polymeric plasticisers such as polyester, polyadipates, polyphosphates, ... .

The compounds of the present invention are useful as stabilisers of polymers of vinyl chloride, vinyl resins containing vinyl chloride units in the structure, such as copolymers of vinyl chloride and vinyl esters of aliphatic acids, preferably vinyl acetate, copolymers of vinyl chloride with esters of acrylic and methacrylic acid and with acrylonitrile, copolymers of vinyl chloride with diene compounds and unsaturated dicarboxylic acids or their anhydrides, such as copolymers of vinyl chloride with diethyl maleate, diethyl fumarate or maleic acid anhydride, post-chlorinated polymers and copolymers of vinyl chloride and vinylidene chloride with unsaturated aldehydes and ketones, such as acrolein, crotonaldehyde, vinyl methyl ketone, vinylmethylether, vinyl isobuthylether and the like; polymers of vinylidene chloride and copolymers thereof with vinyl chlorides or other polymerisable compounds; polymers of vinyl chloroacetate and dichlorovinyl ether; chlorinated polymers of vinyl acetate, chlorinated polymeric esters of acrylic acid and of α-substituted acrylic acid; polymers of chlorinated styrene; chlorinated rubbers; chlorinated polymers of ethylene; polymers and post-chlorinated polymers of chlorobutadiene and their copolymers with vinyl chloride, chlorinated rubber; mixtures of the cited polymers alone or with other polymerisable compounds.

These materials also include graft polymers of PVC with EVA, ABS and MBS. Preferred substrates are also mixtures of above homo and copolymers, preferably homopolymers of vinyl chloride with other thermoplastic or elastomeric polymers, in particular blends with ABS, MBS, NBR, SAN, EVA, CPE, MBAS, PMA, PMMA, EPDM and polylactones.

Suitable stabilisable compositions within the scope of this invention, are preferably recycled chlorine containing polymers; these polymers are the above mentioned polymers which have suffered damage through use, storage or production process. Particularly preferred is recycled PVC. Recycled products may also contain minor amounts of foreign material difficult to remove, such as paper, pigments, adhesives.

According to a fundamental aspect of the present invention the presence of a phenate or carboxylate group of a metal cation of the II group of the periodic table in the aminouracyls used as PVC stabilisers permits to obtain a good transparency of the stabilised system, without staining and blistering, even in the absence of metal soaps.

Some preparations of functionalised aminouracyls according to the invention are here below reported, together with their application tests.

### Example 1 (comparative)

N-(3-hydroxyphenyl)-N'-n-butyl-urea.

A solution of 23.25 g (275 mmoli) of n-butyl isocyanate in 100 ml of acetone was added dropwise, in 35 minutes, into 30.00 g (275 mmoli) of 3-hydroxy aniline dissolved in 200 ml of acetone under stirring at a temperature below 15 °C. The reaction mixture was stirred for 2 hours.

Then the acetone was evaporated under vacuum and the solid washed with water , filtered and dried under vacuum, 50.65 g (88.5 %) of a white solid were obtained.
IR: (cm⁻¹): 3381, 3314, 2961, 2928, 2862, 1691, 1664, 1593, 1576.
¹HNMR (Acetone d6): δ (ppm) 0.80 (t, 3H); 1.34 (m, 2H); 1.45 (m, 2H); 3.83 (m, 2H); 6.39 (d, 1H); 6.76 (d, 1H); 6.98 (t, 1H); 7.24 (s,1H).

### Example 2 (comparative)

6-amino-3-n-butyl-1-(3-hydroxyphenyl)- and 6-amino-3-(3-hydroxyphenyl)-1-n-butyl-uracyl.

30.00 g (144.2 mmoli) of N-(3-hydroxyphenyl)-N'-n-butyl-urea were mixed under stirring with 25.76 g (303.1 mmoli) of cyanacetic acid and 30.90 g (302.9 mmoli) of acetic anhydride.

The mixture was mantained at 95 °C for 3.5 hours, then 200 ml of water and a 15% sodium hydroxide solution were added to the reaction mixture to obtain a pH of 9-10, then the reaction mixture is refluxed for 2 hours.

After cooling at room temperature acetic acid was added obtaining a precipitate. The precipitate was filtered off , washed in ethyl acetate and dried under vacuum obtaining 23.52 g (59.3 %) of solid.
IR: (cm⁻¹): 3467, 3346, 3240, 2960, 2928, 2873, 1675, 1650, 1626.
¹HNMR (DMSO d6): δ (ppm): 0.79 (t, 3H); 1.27 (m, 2H); 1.49 (m, 2H); 3.73 (m, 2H); 4.79 (s, 1H); 6.65 (s, 1H); 6.70 (d, 1H); 6.89 (d, 1H); 7.32 (t, 1H).
¹³CNMR: (DMSO d6): δ (ppm): 13.2, 19.2, 29.3, 74.6, 116.0, 129.2, 130.0, 134.9, 150.8, 153.8, 157.9, 161.1.
MS: 276 (M+1)

### Example 3

Bis-[6-amino-3-n-butyl-1-(3-hydroxyphenyl)- and 6-amino-3-(3-hydroxyphenyl)-1-n-butyl-uracyl] calcium salt.

18 g (65.45 mmoli) of 6-amino-3-n-butyl-1-(3-hydroxyphenyl)- and 6-amino-3-(3-hydroxyphenyl)-1-n-butyl-uracyl were added into 250 cc of water and vigorously stirred at a temperature of 50 °C.

After the addition of 3.04 g (32.86 mmoli) of calcium hydroxide (80%) the reaction mixture was stirred for 2 hours.

The aqueous solution was treated with carbon and filtered, then evaporated under vacuum. Acetone was added to obtain a precipitate. The solid was collected by filtration and dried obtaining 17.68 g (91.9 %) of solid.
¹HNMR (CDCl₃-DMSO d6): δ (ppm): 0.86 (t, 3H); 1.26 (m,2H); 1.49 (m, 2H); 3.80 (m, 2H); 4.78 (s, 1H); 6.14 (bm, 1H); 6.43 (s, 1H); 6.67 (bm,1H); 6.94 (bm, 1H).
MS: 276 (M+1)
MS: 274 (M-1), 549 [2(M-1) + H⁺], 823 [3(M-1) + 2H⁺], 862 [3(M-1) + CA⁺⁺]

### Example 4 (comparative)

N-(3-carboxyphenyl)-N'-n-butyl-urea.

A solution of 3.53 g (35.67 mmoli) of n-butyl isocyanate in 150 ml of acetone were added dropwise, in 20 minutes, to a solution of 5 g (36.39 mmoli) of 3-aminobenzoic acid in 300 ml of acetone under stirring at 50 °C.

After 1 hour the acetone was evapored under vacuum and the solid washed with water , filtered and dried under vacuum. 6.53 g (77.5 %) of white solid was obtained.
IR: (cm⁻¹): 3351, 3286, 2959, 2930, 2860, 1690, 1630, 1592, 1569.
¹HNMR (DMSO d6): δ (ppm): 0.91 (t,3H); 1.33 (m,2H); 1.44 (m,2H); 3.21 (m,2H); 7.32 (t,1H); 7.47 (d,1H); 7.58 (d,1H); 8.03 (s,1H).
MS: 237 (M+1)

### Example 5 (comparative)

6-amino-3-n-butyl-1-(3 carboxyphenyl)- and 6-amino-3-(3 carboxyphenyl)-1-n-butyl -uracyl.

1 g (4.23 mmoli) of N-(3-carboxyphenyl)-N'-n-butyl-urea were added under stirring to 0.738 g (8.68 mmoli) of cyanacetic acid and 0.454 g (4.45 mmoli) of acetic anhydride.

The mixture was heated at 90 °C for 3.5 hours then cooled to room temperature, diluted with water and washed with diethyl ether. The aqueous phase was heated at 50 °C and sodium bicarbonate was added up to pH 7.

After 2 hours under stirring acetic acid was added to pH 4-5, then the organic was extracted with dichloromethane.

The solvent was evaporated under vacuum to obtain 0.44 g (34.3 %) of a white solid.
IR: (cm⁻¹): 3387, 3317, 3252, 3214, 2958, 2873, 1687, 1649, 1632, 1588.
¹HNMR (DMSO d6): δ (ppm): 0.89 (t,3H); 1.27 (m,2H); 1.51 (m,2H); 3.73 (t,2H); 4.83 (s,1H); 7.57 (d,1H); 7.65 (t,1H); 7.81 (s,1H); 8.05 (d,1H).
MS: 304 (M+1)

### Example 6.

Bis-[6-amino-3-n-butyl-1-(3-carboxyphenyl)- and 6-amino-3-(3-carboxyphenyl)-1-n-butyl-uracyl] calcium salt.

A mixture of 0.36 g (1.19 mmoli) of 6-amino-3-n-butil-1-(3-carboxyphenyl)- and 6-amino-3-(3-carboxyphenyl)-1-n-butyl-uracyl and 0.055 g (0.59 mmoli) of calcium hydroxide (80%) in 15 ml of water was stirred at 40 °C for 15 minutes.

The solution was filtered and evaporated under vacuum. Acetone was added to the residue obtaining a precipitate which was filtered off and dried under vaacum, 0.2 g (56.7 %) of white solid are obtained.
¹HNMR.(DMSO d6): δ (ppm):0.85 (t,3H); 1.25 (m,2H); 1.45 (m,2H); 3.70 (t,2H); 4.80 (s,1H); 7.23 (d,1H); 7.42 (t,1H); 7.67 (s,1H); 7.98 (d,1H).
Ms: 302 (M-1)

### Application tests - Static heat tests.

The thermal stability of PVC compositions stabilised with the products of the present invention is measured as reported below.

A dry mixture of the ingredients reported in the following tables, is laminated using a two cylinder apparatus for 2 min at 200°C. From the obtained rolled sheet, 20 x 220 mm samples are cut. The test sample is subject to thermal stress in a Wherner Mathis oven equipped with a thermotester device. The oven temperature is set at 190°C and the thermotester device is set so that 20 mm of test sample are removed from the oven at regular intervals. In the reported conditions the sample is under a static heat stress. At the end of the experiment the evaluation is carried out measuring the White Index (L* value of the chromatic co-ordinates of the CIE L*a*b* system) according to ASTM D1925-70 standard method.

The compound of Example 3 is compared with Reference Compound 6-amino-1,3-dimethyl uracyl (6-amino-1,3-dimethyl-1H-pyrimidine-2,4-dione).

The results on rigid and flexible PVC are reported below.

High values of L* signify a good stabilisation.

**Table I Compositions (numbers refer to parts by weight)**

| **Composition** | **Comp.1*** | **Comp.2*** | **Comp.3** |
|---|---|---|---|
| S-PVC (k value 68) | 100 | 100 | 100 |
| Stearic acid | 0.5 | 0.5 | 0.5 |
| Reference Compound | - | 0.4 | - |
| Compound of Example 3 | - | - | 0.4 |

| | | | |
|---|---|---|---|
| *: comparative | | | |

**Table II - Static heat test**

| **Thermal stress duration (min.)** | **L* Comp. 1*** | **L* Comp.2*** | **L* Comp.3** |
|---|---|---|---|
| 3' | 54.12 | 65.52 | 75.12 |
| 6' | Burning | 60.27 | 75.51 |
| 9' | | 44.71 | 75.11 |
| 12' | | 38.28 | 65.90 |
| 15' | | 28.94 | 35.66 |
| 18' | | Burning | 25.65 |
| 21' | | | 22.97 |
| 24' | | | Burning |

| | | | |
|---|---|---|---|
| *: comparative | | | |

**Table III Compositions (numbers refer to parts by weight)**

| **Compostions** | **Comp.4*** | **Comp.5*** | **Comp.6** | **Comp.7** | **Comp.8** |
|---|---|---|---|---|---|
| S-PVC (k value 68) | 100 | 100 | 100 | 100 | 100 |
| Calcium carbonate 5 microns | 3 | 3 | 3 | 3 | 3 |
| Stearic acid | 0.5 | 0.5 | 0.5 | 0.5 | 0.5 |
| Reference Compound | - | 0.4 | - | - | - |
| Compound of Example 3 | - | - | 0.4 | 0.6 | 0.8 |

| | | | | | |
|---|---|---|---|---|---|
| *: comparative | | | | | |

**Table IV - Static heat test**

| **Thermal stress duration (min.)** | **L* Comp.4*** | **L* Comp.5*** | **L* Comp.6** | **L* Comp.7** | **L* Comp.8** |
|---|---|---|---|---|---|
| 3' | 31.25 | 61.94 | 74.89 | 78.22 | 79.54 |
| 6' | Burning | 56.97 | 72.79 | 76.89 | 77.94 |
| 9' | | 44.37 | 69.03 | 73.59 | 75.12 |
| 12' | | 34.58 | 61.46 | 67.57 | 69.05 |
| 15' | | 24.44 | 35.58 | 62.26 | 64.57 |
| 18' | | Burning | 26.33 | 49.18 | 53.91 |
| 21' | | | | 34.62 | 46.40 |
| 24' | | | | 25.6 | 35.12 |

| | | | | | |
|---|---|---|---|---|---|
| *: comparative | | | | | |

**Table V: Compositions (numbers refer to parts by weight)**

| **Compostions** | **Comp.9*** | **Comp.10*** | **Comp.11** | **Comp.12** | **Comp.13** |
|---|---|---|---|---|---|
| S-PVC (k value 70) | 100 | 100 | 100 | 100 | 100 |
| DOP** | 60 | 60 | 60 | 60 | 60 |
| Stearic acid | 0.5 | 0.5 | 0.5 | 0.5 | 0.5 |
| Reference Compound | - | 0.4 | - | - | - |
| Compound of Example 3 | - | - | 0.4 | 0.6 | 0.8 |

| | | | | | |
|---|---|---|---|---|---|
| *: comparative | | | | | |
| ** dioctyl phthalate | | | | | |

**Table VI - Static heat test**

| **Thermal stress duration (min.)** | **L* Comp.9*** | **L* Comp.10*** | **L* Comp.11** | **L* Comp.12** | **L* Comp.13** |
|---|---|---|---|---|---|
| 3' | 46.42 | 80.51 | 87.98 | 88.10 | 88.95 |
| 6' | 29.3 | 73.86 | 85.82 | 86.68 | 87.86 |
| 9' | Burning | 71.97 | 85.08 | 86.18 | 87.05 |
| 12' | | 68.23 | 84.41 | 85.19 | 86.54 |
| 15' | | 63.44 | 84.10 | 84.78 | 85.68 |
| 18' | | 55.80 | 81.51 | 82.36 | 83.05 |
| 21' | | 41.90 | 57.16 | 80.22 | 82.54 |
| 24' | | 32.45 | 35.09 | 46.61 | 80.12 |

**Table VII: Compositions (numbers refer to parts by weight)**

| **Compostions** | **Comp.14*** | **Comp.15*** | **Comp.16** |
|---|---|---|---|
| S-PVC (k value 70) | 100 | 100 | 100 |
| DOP** | 60 | 60 | 60 |
| Stearic acid | 0.5 | 0.5 | 0.5 |
| Epoxidised soy-bean oil*** | 2 | 2 | 2 |
| Reference Compound | - | 0.4 | - |
| Compound of Example 3 | - | - | 0.4 |

| | | | |
|---|---|---|---|
| *: comparative | | | |
| **dioctyl phthalate | | | |
| *** oxyranic oxygen >6g(O₂)/100g | | | |

**Table VIII- Static heat test**

| **Thermal stress duration (min.)** | **L* Comp.14*** | **L* Comp.15*** | **L* Comp.16** |
|---|---|---|---|
| 5' | 86.58 | 85.35 | 87.93 |
| 10' | 84.86 | 78.85 | 83.22 |
| 15' | 82.50 | 73.24 | 85.38 |
| 20' | 64.38 | 72.33 | 84.48 |
| 25' | 47.94 | 70.05 | 82.79 |
| 30' | 41.16 | 68.57 | 81.21 |
| 35' | 37.84 | 66.53 | 75.10 |
| 40' | 34.67 | 57.66 | 67.34 |
| 45' | 29.32 | 56.04 | 63.88 |
| 50' | | 48.68 | 60.61 |

- Evaluation of staining and blistering.

The appraisal of staining and blistering is carried out by the evaluation, respectively, of exudation and transparency on test samples obtained from lamination using a two cylinder apparatus for 1 min at 190°C.

The exudation is evaluated by an organoleptic test. An exudated sample is tacky. An empirical evaluation has been set up with scores from 0 to 3, according to the definitions of Table IX.

**Table IX**

| **Degree of exudation** | **Tacky of the test sample** |
|---|---|
| 0 | Tack free |
| 1 | Lightly tacky |
| 2 | Moderately tacky |
| 3 | Strongly tacky |

Transparency is measured by a BYK Colour Guide 45/0 colorimeter using the setting to measure opacity.

The test sample is placed on a white and black cardboard and two different measurements are carried out: one against the black background and the other against the white background: opacity is the ratio % between the evaluation against the black background and the white background.

The higher is the %, the higher is opacity and the lower is transparency.

The results of the application tests for the evaluation of exudation and transparency on flexible PVC are reported.

The comparison is made among Compositions 17, Composition 18 and a similar composition of flexible PVC containing the Reference Compound 6-amino-1,3-dimethyl uracyl (Composition 19).

**Table X: Compositions (numbers refer to parts by weight)**

| **Compostions** | **Comp.17*** | **Comp.18** | **Comp.19*** |
|---|---|---|---|
| S-PVC (k value 70) | 100 | 100 | 100 |
| DOP** | 60 | 60 | 60 |
| Stearic acid | 0.2 | 0.2 | 0.2 |
| Epoxidised soy-bean oil*** | 2 | 2 | 2 |
| Compound of Example 3 | - | 0.4 | - |
| Reference Compound | - | - | 0.4 |

| | | | |
|---|---|---|---|
| *: comparative | | | |
| **dioctyl phthalate | | | |
| *** oxyranic oxygen >6g(O₂)/100g | | | |

**Table XI - Exudation and transparency**

| **Composition** | **Exudation value** | **Transparency %** |
|---|---|---|
| 17* | 0 | 16.1 |
| 18 | 0 | 17.4 |
| 19* | 3 | 21.3 |

| | | |
|---|---|---|
| *: comparative | | |

## Claims

1. PVC compositions containing aminouracyls functionalised with a phenate or carboxylate group and having formula I: 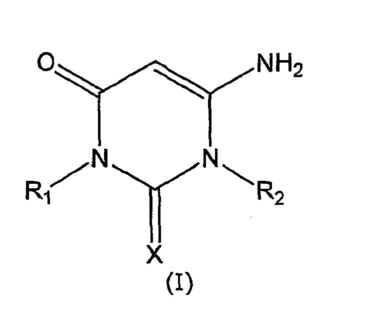 wherein:
X is O or S;
R₁ is a group of formula II: 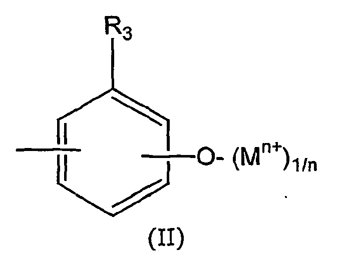
or R₁ is a group of formula III: 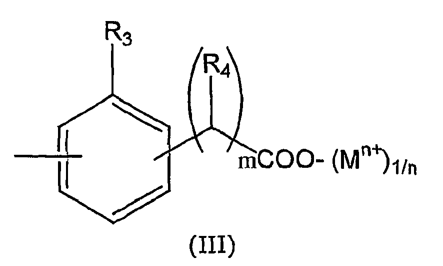
or R₁ is a group of formula IV: 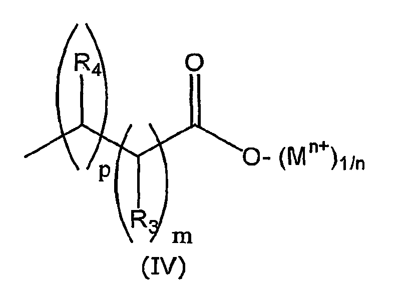 wherein Mⁿ⁺ is a metal cation of the II group of the periodic table or is Zn⁺⁺;
R₃ and R₄ can be the same or different and each independently of the other is selected from the group consisting of -H, C₁-C₆ linear o branched alkyl, -OH, C₁-C₆ linear or branched alkoxyl, halogen atom, -SH, C₁-C₆ linear or branched thioalkoxyl, NR₅R₆ (wherein R₅ and R₆ can be equal or different and are H or C₁-C₆ linear o branched alkyl), nitro, carboxyl, C₁-C₆ linear or branched carboxyalkyl;
m and p are integers having a value of from 0 to 6, with the proviso that they have not together the value of 0;
R₂ is the same as R₁ or it is selected in the group consisting of C₁-C₁₈ linear or branched alkyl, C₁-C₁₈ linear or branched alkyl substituted with -OH, halogen atom, C₁-C₆ linear or branched carboxyalkyl, -SH, C₁-C₆ linear or branched thioalkoxyl, NR₅R₆ (wherein R₅ and R₆ can be equal or different and are H or C₁-C₆ linear o branched alkyl), C₁-C₆ linear or branched carboxyalkyl.

2. PVC compositions according to claim 1., wherein M is selected among Ca, Ba and Zn and n is 2.

3. PVC compositions according to claim 2., wherein R₂ is a C₁-C₁₈ linear or branched alkyl.

4. PVC compositions according to claim 3., wherein R₂ is n-butyl.

5. PVC compositions according to claim 3. or 4., wherein R₁ is a group of formula II and R₃ is H.

6. PVC compositions according to claim 3. or 4., wherein R₁ is a group of formula III, m=0 and R₃ is H.

7. PVC compositions according to claim 3. or 4., wherein R₁ is a group of formula IV and R₄ and R₃ are H.

8. PVC compositions according to any of the preceding claims, containing from 0.01 to 10% by weight of aminouracyls.

9. PVC compositions according to claim 8., containing from 0.05 to 5% by weight of aminouracyls.

10. PVC compositions according to claim 9., containing from 0.1 to 3% by weight of aminouracyls.

11. Aminouracyls functionalised with a phenate or carboxylate group and having formula I: 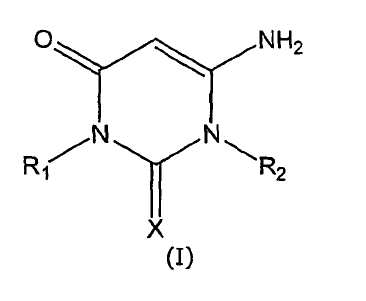 wherein:
X is O or S;
R₁ is a group of formula II: 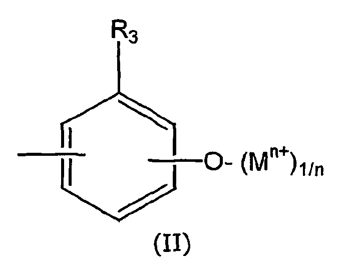
or R₁ is a group of formula III: 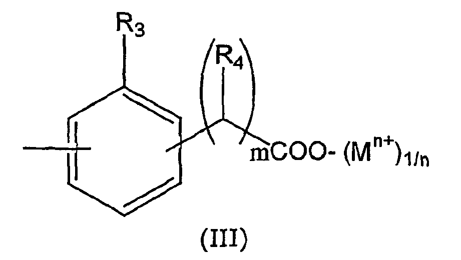
or R₁ is a group of formula IV: 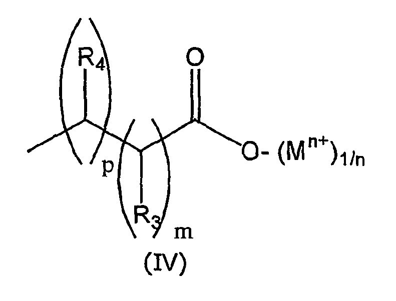 wherein Mⁿ⁺ is a metal cation of the II group of the periodic table or is Zn⁺⁺;
R₃ and R₄ can be the same or different and each independently of the other is selected from the group consisting of -H, C₁-C₆ linear o branched alkyl, -OH, C₁-C₆ linear or branched alkoxyl, halogen atom, -SH, C₁-C₆ linear or branched thioalkoxyl, NR₅R₆ (wherein R₅ and R₆ can be equal or different and are H or C₁-C₆ linear o branched alkyl), nitro, carboxyl, C₁-C₆ linear or branched carboxyalkyl;
m and p are integers having a value of from 0 to 6, with the proviso that they have not together the value of 0;
R₂ is the same as R₁ or it is selected in the group consisting of C₁-C₁₈ linear or branched alkyl, C₁-C₁₈ linear or branched alkyl substituted with -OH, halogen atom, C₁-C₆ linear or branched carboxyalkyl, -SH, C₁-C₆ linear or branched thioalkoxyl, NR₅R₆ (wherein R₅ and R₆ can be equal or different and are H or C₁-C₆ linear o branched alkyl), C₁-C₆ linear or branched carboxyalkyl.

12. Aminouracyls functionalised with a phenate or carboxylate group according to claim 11., wherein M is selected among Ca, Ba and Zn and n is 2.

13. Aminouracyls functionalised with a phenate or carboxylate group according to claim 12., wherein R₂ is a C₁-C₁₈ linear or branched alkyl.

14. Aminouracyls functionalised with a phenate or carboxylate group according to claim 13., wherein R₂ is n-butyl.

15. Aminouracyls functionalised with a phenate or carboxylate group according to claim 13. or 14., wherein R₁ is a group of formula II and R₃ is H.

16. Aminouracyls functionalised with a phenate or carboxylate group according to claim 13. or 14., wherein R₁ is a group of formula III, m=0 and R₃ is H.

17. Aminouracyls functionalised with a phenate or carboxylate group according to claim 13. or 14., wherein R₁ is a group of formula IV and R₄ and R₃ are H.

## Patentansprüche

1. PVC-Zusammensetzungen, die Aminouracile enthalten und mit einer Phenolat- oder Carboxylatgruppe funktionalisiert wurden und die die Formel I besitzen: 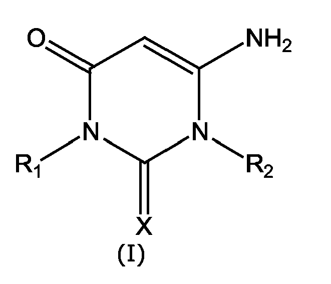 worin:
X ein O oder S ist;
R₁ eine Gruppe der Formel II ist: 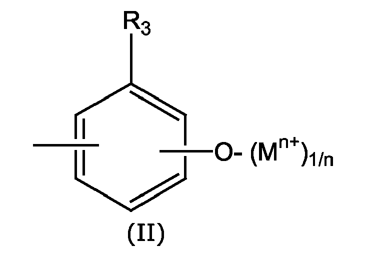
oder R₁ eine Gruppe der Formel III ist: 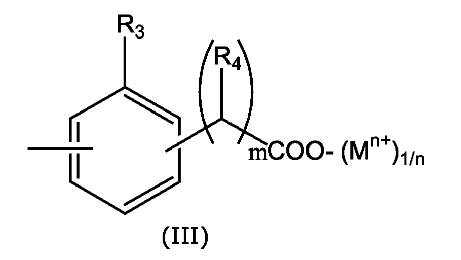
oder R₁ eine Gruppe der Formel IV ist: 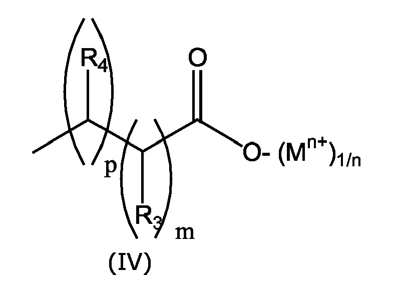 worin Mⁿ⁺ ein metallisches Kation der II. Gruppe des Periodensystems oder Zn⁺⁺ ist;
R₃ und R₄ gleich oder unterschiedlich sein können und beide unabhängig voneinander von der Gruppe gewählt werden können, die aus einem -H, C₁-C₆ linearem oder verzweigtem Alkyl, einem -OH, einem C₁-C₆ linearen oder verzweigten Alkoxyl, einem Halogenatom, einem -SH, einem C₁₋C₆ linearen oder verzweigten Thioalkoxyl, einem NR₅R₆ (worin R₅ und R₆ gleich oder unterschiedlich sein können und ein H oder ein C₁-C₆ lineares oder verzweigtes Alkyl ist), einem Nitro, einem Carboxyl, einem C₁-C₆ linearem oder verzweigten Carboxyalkyl besteht;
m und p ganze Zahlen sind mit einem Wert zwischen 0 und 6, unter der Bedingung, dass diese nicht gemeinsam den Wert 0 aufweisen;
R₂ gleich R₁ ist oder in der Gruppe ausgewählt wird, die aus einem C₁₋C₁₈ linearen oder verzweigten Alkyl, einem C₁-C₁₈ linearen oder verzweigten Alkyl besteht, das mit einem -OH, einem Halogenatom, einem C₁-C₆ linearen oder verzweigten Carboxyalkyl, einem -SH, einem C₁-C₆ linearen oder verzweigten Thioalkoxyl, einem NR₅R₆ (worin R₅ und R₆ gleich oder unterschiedlich sein können und ein H oder ein C₁-C₆ lineares oder verzweigtes Alkyl sind), einem C₁-C₆ linearen oder verzweigten Carboxyalkyl substituiert wurde.

2. PVC-Zusammensetzungen entsprechend Patentanspruch 1., worin M aus einem Ca, Ba und Zn ausgewählt wird und n gleich 2 ist.

3. PVC-Zusammensetzungen entsprechend Patenanspruch 2., worin R₂ ein C₁-C₁₈ lineares oder verzweigtes Alkyl ist.

4. PVC-Zusammensetzungen entsprechend Patentanspruch 3., worin R₂ ein n-Butyl ist.

5. PVC-Zusammensetzungen entsprechend 3. oder 4., worin R₁ eine Gruppe der Formel II und R₃ ein H ist.

6. PVC-Zusammensetzungen entsprechend Patentanspruch 3. oder 4., worin R₁ eine Gruppe der Formel III, m=0 und R₃ ein H ist.

7. PVC-Zusammensetzungen entsprechend Patentanspruch 3. oder 4., worin R₁ eine Gruppe der Formel IV und R₄ und R₃ ein H ist.

8. PVC-Zusammensetzungen entsprechend jeder der vorangehenden Patentansprüche, die zwischen 0.01 und 10% Gewichtsanteil an Aminouracilen enthalten.

9. PVC-Zusammensetzungen entsprechend Patentanspruch 8., die zwischen 0.05 und 5% Gewichtsanteil an Aminouracilen enthalten.

10. PVC-Zusammensetzungen entsprechend Patentanspruch 9., die zwischen 0.1 und 3% Gewichtsanteil an Aminouracilen enthalten.

11. Aminouracile, die mit einer Phenolat- oder Carboxylatgruppe funktionalisiert wurden und die die Formel I besitzen: 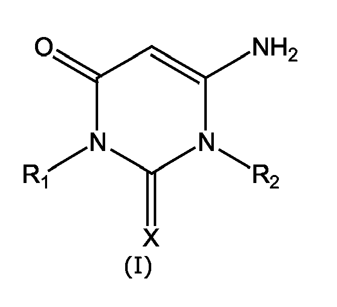 worin:
X ein O oder S ist;
R₁ eine Gruppe der Formel II ist: 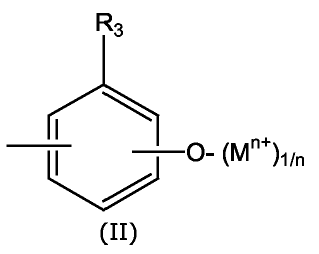
oder R₁ eine Gruppe der Formel III ist: 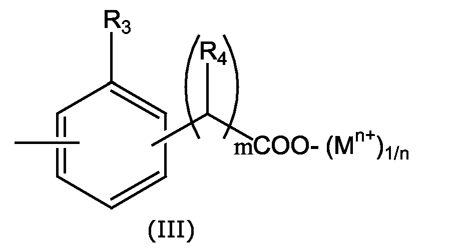
oder R₁ eine Gruppe der Formel IV ist: 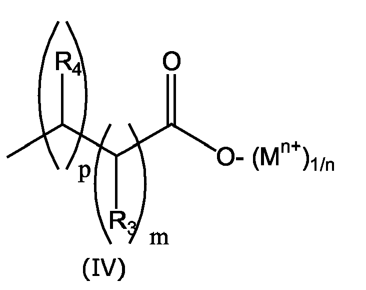 worin Mⁿ⁺ ein metallisches Kation der II. Gruppe des Periodensystems oder Zn⁺⁺ ist;
R₃ und R₄ gleich oder unterschiedlich sein können und beide unabhängig voneinander von der Gruppe gewählt werden können, die aus einem -H, einem C₁-C₆ linearem oder verzweigtem Alkyl, einem -OH, einem C₁-C₆ linearen oder verzweigten Alkoxyl, einem Halogenatom, einem -SH, einem C₁-C₆ linearen oder verzweigten Thioalkoxyl, einem NR₅R₆ (worin R₅ und R₆ gleich oder unterschiedlich sein können und ein H oder ein C₁₋C₆ lineares oder verzweigtes Alkyl sind), einem Nitro, einem Carboxyl, einem C₁-C₆ linearem oder verzweigten Carboxyalkyl besteht;
m und p ganze Zahlen sind mit einem Wert zwischen 0 und 6, unter der Bedingung, dass diese nicht gemeinsam den Wert 0 aufweisen;
R₂ gleich R₁ ist oder in der Gruppe ausgewählt wird, die aus einem C₁₋C₁₈ linearen oder verzweigten Alkyl, einem C₁-C₁₈ linearen oder verzweigten Alkyl besteht, das mit einem -OH, einem Halogenatom, einem C₁-C₆ linearen oder verzweigten Carboxyalkyl, einem -SH, einem C₁-C₆ linearen oder verzweigten Thioalkoxyl, einem NR₅R₆ (worin R₅ und R₆ gleich oder unterschiedlich sein können und ein H oder ein C₁-C₆ lineares oder verzweigtes Alkyl sind), einem C₁-C₆ linearen oder verzweigten Carboxyalkyl substituiert wurde.

12. Aminoruacile, die mit einer Phenolat- oder Carboxylatgruppe funktionalisiert wurden entsprechend Patentanspruch 11., worin M aus Ca, Ba und Zn ausgewählt wird und n gleich 2 ist.

13. Aminoruacile, die mit einer Phenolat- oder Carboxylatgruppe funktionalisiert wurden entsprechend Patentanspruch 12., worin R₂ ein C₁-C₁₈ lineares oder verzweigtes Alkyl ist.

14. Aminoruacile, die mit einer Phenolat- oder Carboxylatgruppe funktionalisiert wurden entsprechend Patentanspruch 13., worin R₂ ein n-Butyl ist.

15. Aminoruacile, die mit einer Phenolat- oder Carboxylatgruppe funktionalisiert wurden entsprechend Patentanspruch 13. oder 14., worin R₁ eine Gruppe der Formel II und R₃ ein H ist.

16. Aminoruacile, die mit einer Phenolat- oder Carboxylatgruppe funktionalisiert wurden entsprechend Patentanspruch 13. oder 14., worin R₁ eine Gruppe der Formel III, m=0 und R₃ ein H ist.

17. Aminoruacile, die mit einer Phenolat- oder Carboxylatgruppe funktionalisiert wurden entsprechend Patentanspruch 13. oder 14., worin R₁ eine Gruppe der Formel IV und R₄ und R₃ ein H ist.

## Revendications

1. Compositions de PVC contenant des aminouracyles fonctionnalisés par un groupe phénate ou carboxylate et ayant la formule I : 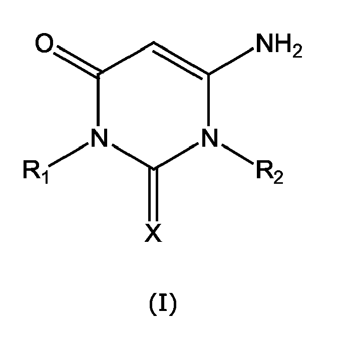 dans laquelle :
X est O ou S ;
R₁ est un groupe de formule II : 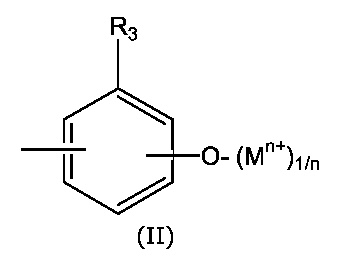
ou R₁ est un groupe de formule III : 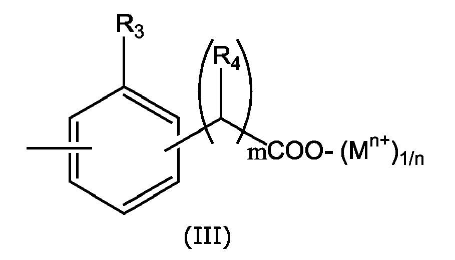
ou R₁ est un groupe de formule IV : 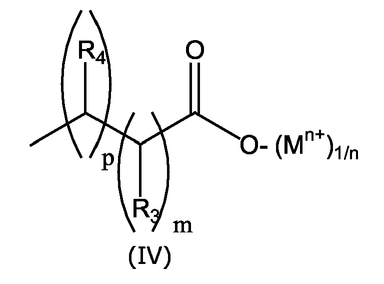 dans lesquelles Mⁿ⁺ est un cation d'un métal du groupe II du tableau périodique ou est Zn⁺⁺ ;
R₃ et R₄ peuvent être les mêmes ou différents et chacun, indépendamment l'un de l'autre, est choisi dans le groupe formé des groupes -H, alkyle linéaire ou ramifié en C₁ à C₆, -OH, alcoxyle linéaire ou ramifié en C₁ à C₆, atome d'halogène, -SH, thioalcoxyle linéaire ou ramifié en C₁ à C₆, NR₅R₆ (où R₅ et R₆ peuvent être égaux ou différents et sont H ou un alkyle linéaire ou ramifié en C₁ à C₆), nitro, carboxyle, carboxyalkyle linéaire ou ramifié en C₁ à C₆ ;
m et p sont des entiers ayant une valeur de 0 à 6, à la condition qu'ils n'aient pas en même temps la valeur 0 ;
R₂ est identique à R₁ ou il est choisi dans le groupe formé des groupes alkyle linéaire ou ramifié en C₁ à C₁₈ , alkyle linéaire ou ramifié en C₁ à C₁₈ à substitution -OH, atome d'halogène, carboxyalkyle linéaire ou ramifié en C₁ à C₆ , -SH, thioalcoxyle linéaire ou ramifié en C₁ à C₆, NR₅R₆ (où R₅ et R₆ peuvent être égaux ou différents et sont H ou un alkyle linéaire ou ramifié en C₁ à C₆), carboxyalkyle linéaire ou ramifié en C₁ à C₆.

2. Compositions de PVC selon la revendication 1., où M est choisi parmi Ca, Ba et Zn et n est égal à 2.

3. Compositions de PVC selon la revendication 2., où R₂ est un alkyle linéaire ou ramifié en C₁ à C₁₈.

4. Compositions de PVC selon la revendication 3, où R₂ est le n-butyle.

5. Compositions de PVC selon la revendication 3. ou 4., où R₁ est un groupe de formule II et R₃ est H.

6. Compositions de PVC selon la revendication 3. ou 4., où R₁ est un groupe de formule III, m = 0 et R₃ est H.

7. Compositions de PVC selon la revendication 3. ou 4., où R₁ est un groupe de formule IV et R₄ et R₃ sont H.

8. Compositions de PVC selon l'une quelconque des revendications précédentes, contenant de 0,01 à 10 % en poids d'aminouracyles.

9. Compositions de PVC selon la revendication 8., contenant de 0,05 à 5 % en poids d'aminouracyles.

10. Compositions de PVC selon la revendication 9., contenant de 0,1 à 3 % en poids d'aminouracyles.

11. Aminouracyles fonctionnalisés par un groupe phénate ou carboxylate et de formule I : 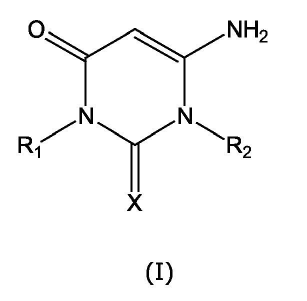 dans laquelle :
X est O ou S ;
R₁ est un groupe de formule II : 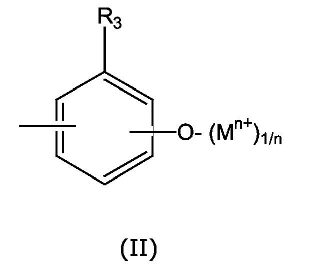
ou R₁ est un groupe de formule III : 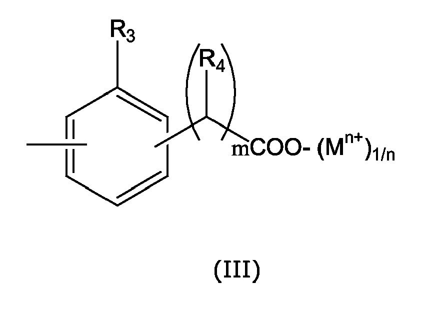
ou R₁ est un groupe de formule IV : 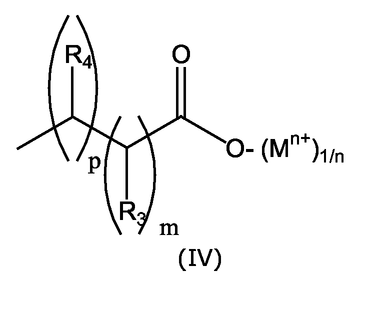 dans lesquelles Mⁿ⁺ est un cation d'un métal du groupe II du tableau périodique ou est Zn⁺⁺ ;
R₃ et R₄ peuvent être les mêmes ou différents et chacun, indépendamment de l'autre, est choisi dans le groupe formé des groupes -H, alkyle linéaire ou ramifié en C₁ à C₆ , -OH, alcoxyle linéaire ou ramifié en C₁ à C₆ , atome d'halogène, -SH, thioalcoxyle linéaire ou ramifié en C₁ à C₆ , NR₅R₆ (où R₅ et R₆ peuvent être égaux ou différents et sont H ou un alkyle linéaire ou ramifié en C₁ à C₆), nitro, carboxyle, carboxyalkyle linéaire ou ramifié en C₁ à C₆ ;
m et p sont des entiers ayant une valeur de 0 à 6, à la condition qu'ils n'aient pas en même temps la valeur 0 ;
R₂ est identique à R₁ ou il est choisi dans le groupe formé des groupes alkyle linéaire ou ramifié en C₁ à C₁₈ , alkyle linéaire ou ramifié en C₁ à C₁₈ à substitution -OH, atome d'halogène, carboxyalkyle linéaire ou ramifié en C₁ à C₆ , -SH, thioalcoxyle linéaire ou ramifié en C₁ à C₆, NR₅R₆ (où R₅ et R₆ peuvent être égaux ou différents et sont H ou un alkyle linéaire ou ramifié en C₁ à C₆), carboxyalkyle linéaire ou ramifié en C₁ à C₆.

12. Aminouracyles fonctionnalisés par un groupe phénate ou carboxylate selon la revendication 11., où M est choisi parmi Ca, Ba et Zn et n est égal à 2.

13. Aminouracyles fonctionnalisés par un groupe phénate ou carboxylate selon la revendication 12., où R₂ est un alkyle linéaire ou ramifié en C₁ à C₁₈.

14. Aminouracyles fonctionnalisés par un groupe phénate ou carboxylate selon la revendication 13., où R₂ est le n-butyle.

15. Aminouracyles fonctionnalisés par un groupe phénate ou carboxylate selon la revendication 13. ou 14., où R₁ est un groupe de formule II et R₃ est H.

16. Aminouracyles fonctionnalisés par un groupe phénate ou carboxylate selon la revendication 13. ou 14., où R₁ est un groupe de formule III, m=0 et R₃ est H.

17. Aminouracyles fonctionnalisés par un groupe phénate ou carboxylate selon la revendication 13. ou 14., où R₁ est un groupe de formule IV et R₄ et R₃ sont H.
